(19) Europäisches Patentamt
European Patent Office
Office européen des brevets

(11) Publication number: **0 289 110**
**A2**

(12) **EUROPEAN PATENT APPLICATION**

(21) Application number: **88300813.8**

(51) Int. Cl.⁴: **A61K 39/385**

(22) Date of filing: **01.02.88**

Claims 32 + 36 are deemed to be abandoned due to non-payment of the claims fees (Rule 31 (2) EPC).

(30) Priority: **02.02.87 US 9441**
**29.01.88 US 150359**

(43) Date of publication of application:
**02.11.88 Bulletin 88/44**

(84) Designated Contracting States:
**AT BE CH DE ES FR GB GR IT LI LU NL SE**

(71) Applicant: **Swiss Serum and Vaccine Institute Berne**
**Rehhagstrasse 79**
**CH-3001 Berne(CH)**

(72) Inventor: **Sadoff Jerald C.**
**1622 Kalmia Road N.W.**
**Washington DC 20012(US)**
Inventor: **Cryz Stanley J. Jr.**
**3176**
**Neuenegg(CH)**

(74) Representative: **Bannerman, David Gardner et al**
**Withers & Rogers 4 Dyer's Buildings Holborn**
**London, EC1N 2JT(GB)**

(54) **Conjugate malaria vaccine.**

(57) The present invention relates to the immunogenic preparations of peptides derived from the circumsporozoite protein of Plasmodium falciparum and/or Plasmodium vivax, covalently coupled to carrier proteins suitable for vaccination of humans. The present invention provides means for covalently linking said peptides to carrier proteins via low molecular weight spacer molecules. The invention further provides means for preparing said peptide-protein conjugates and a vaccine derived from said conjugates.

EP 0 289 110 A2

# CONJUGATE MALARIA VACCINE

The subject invention relates to conjugate immunogenic preparations and their method of production. More specifically, the invention relates to a conjugate vaccine comprising the circumsporozoite protein coupled via at least one spacer molecule, to a carrier protein suitable for vaccination of humans.

## BACKGROUND OF THE INVENTION

Malaria, a debilitating and often fatal disease, is caused by infection with parasites of the genu Plasmodium. Despite vigorous efforts by national and international health agencies, malaria continues to be a leading cause of morbidity and mortality in tropical and subtropical areas of the world. Figures available for 1982 show that 54% of the world's population live in areas where malaria is endemic and are therefore at risk to acquiring the disease. It is estimated that there are 25-30 million new cases of malaria reported per year worldwide. In Africa alone, there are 1 million deaths per annum attributed to malaria. These numbers have remained more or less constant for the past decade.

The etiological agents for human malaria are P. falciparum, which accounts for approximately 80% of cases worldwide, and P. vivax. The life cycle of the malaria parasite is complex, undergoing several stages of differentiation in two hosts, the mosquito and humans. The stage which is infectious for humans in termed the sporozoite and is transmitted to humans via the bite of a mosquito. The sporozoite exists briefly in the bloodstream before entering other cells. Primary infection is thought to be through infection of the liver. The organisms, after differentiating, infect red blood cells where they persist as intracellular parasites. When present in this form, the malaria parasite is sequestered away from the immune system which is able to mount only a weak, non-protective antibody response.

Measures aimed at controlling malaria can, in principle, be directed against three targets; i) eradication of the mosquito vector, ii) curing or management of infected persons, and iii) immunization of the population at risk. Insecticides held much initial promise for the control of the anopheline mosquito vector. However, increased resistance to these agents, detrimental environmental impact, cost of their administration, and inaccessibility of breeding areas have combined to greatly limit the effect of insecticide programs on malaria.

Attempts to cure or manage malaria through the use of several antimalarials have recently been hampered by the emergence of drug resistant strains. Resistance to chloroquine, the "drug of choice," is widespread, which is also the case for the number two regimen, which uses sulfonamide pyrimethamine. Recently, a new antimalarial, melfoquine, was introduced. The use of melfoquine is limited by variable sensibility to the drug among P. falciparum from different geographical regions.

The first attempts to evoke active immunity against malaria were initiated in the 1940's when it was shown that immunization with inactivated intact sporozoites from P. gallinaceum provided protection against fowl malaris. It has subsequently been shown that immunization of mice, monkeys, and man with inactivated sporozoites confers protection against challenge with sporozoite-stage parasites. The sporozoite immunogens used in the above studies were mature sporozoites derived from the salivary glands of mosquitos. Although these studies demonstrated that antisporozoite antibody was capable of providing protection against malaria, the use of inactivated sporozoites as an immunogen is not practical considering that i) the isolation of sporozoites from the salivary glands yields only small quantities of parasites, ii) sporozoites cannot be cultivated on synthetic or semi-synthetic medium, and iii) high titers of anti-sporozoite antibody was elicited only following repeated intravenous immunization or the use of potent adjuvants, both approaches being unsuitable for vaccination of humans.

It is now well recognized that the protective antigen expressed by sporozoites is the circumsporozoite protein (CSP) which is localized on the surface of the parasite, Young, J.F. et al, Science, 228, 958 (1985). The genes involved in the synthesis of the CSP of P. falciparum have been cloned and sequenced. The CSP is composed of 41 tetrapeptide repeat units; 37 (ASN-ALA-ASN-PRO) and 4 ((ASN-VAL-ASP-PRO) flanked by 2 regions "shared" by P. falciparum and P. knowlesi. It has been shown that antibody against the 2 shared regions of the CSP has no protective effect. In contrast, antibody to the ASN-ALA-ASN-PRO region was found to react with intact sporozoites and to block their penetration of hepatic cells, Ballou, W.R. et al., Science, 228, 996 (1985).

Based upon these findings, attempts at malaria vaccine development have centered around the production of antigens capable of evoking an antibody response which recognizes the ASN-ALA-ASN-PRO

region of the CSP. One approach has been to use recombinant DNA technology. A gene segment coding for 15 (ASN-ALA-ASN-PRO) plus 1 (ASN-VAL-ASP-PRO) has been synthesized and 2 copies of the gene spliced together. This sequence is termed $R_{32}$. $R_{32}$ has been spliced into a plasmid coding for tetracycline resistance ($TeT_R$). The fused product codes for 32 amino acide of the $TeT_R$ gene product in addition of $R_{32}$ and is termed $R_{32}$-$TeT_{32}$. When used to immunized mice, $R_{32}$-$TeT_{32}$ engenders an antibody response which recognizes the CSP of intact sporozoites and blocks sporozoite penetration of hepatic cells. The presence of the $TeT_{32}$ gene product was essential for the evoking of an immune response. If the $TeT_{32}$ region was omitted, a potent oil-based adjuvant was needed to evoke an immune response. Subsequently, $R_{32}$-$TeT_{32}$ was adsorbed to $A(OH)_3$ (to act as an adjuvant) and graded doses (10 µg to 1,000 µg) were administered by the intramuscular rote to human volunteers. $R_{32}$-$TeT_{32}$ evoked only a weak anti-sporozoite immune response even at the highest dose tests. This poor immune response, coupled with the relatively large quantity of antigen necessary to generate it, precludes the use of $R_{32}$-$TeT_{32}$ as a human vaccine amenable for public health usage.

An alternative approach to engender an anti-sporozoite immune response has been to use artificially synthesized small molecular weight peptides consisting of the ASN-ALA-ASN-PRO repeat unit. Because of their small molecular weight, these peptides are not immunogenic. The peptides have been covalently linked to carrier proteins to generate conjugate vaccines. In one study, peptides (composed of 2 to 4 repeat units of ASN-ALA-ASN-PRO) were coupled to either bovine serum albumin or thyroglobulin using succinimidyl 4(N-maleimidomethyl)cyclohexane-1-carboxylate (SMCC) and m-maleimidobenzoyl N-hydroxysuccinimide ester (MBS) as coupling agents, Ballou, W.R. et al, 1985, supra. These conjugates were capable of eliciting an anti-sporozoite antibody response in mice when administered with a potent adjuvant. The immune antibody was able to block the penetration of liver cells by sporozoites. Again, these conjugates are not suitable for human use due to the following reasons, i) antibody elicited to the carrier protein may react with human proteins sharing common determinants or, in the case of bovine serum albumin, with ingested food stuffs, ii) the 2 coupling agents employed, SMCC and MBS, possess extremely reactive groups which would be expected to react with host tissue, since they would be incorporated into the conjugate, and iii) the immunogenicity of these conjugates in the absence of an adjuvant or with an adjuvant suitable for human use has not been demonstrated. European patent application 166,410, and PCT application numbers U.S. 84/00144, U.S. 85/01733 and PCT U.S. 86/00627 discuss generally conjugating a peptide to a carrier protein. Conjugate vaccines have been made which comprise a Neisseria meningitidis protein linked to polysaccharide, Einhorn et al, The Lancet, August 9, 1986, p. 299. This type of protein, however, has not been liked to a peptide. These and other publications referred to herein and hereby incorporated by reference.

The genes involved in production of the circumsporozite protein of P. vivax have also been identified. The sequence of the repeating unit consists of: GLY:ASP:ARG:ALA:ASP:GLY:GLU:PRO:ALA.

## SUMMARY OF THE INVENTION

The present invention is related to immunogenic preparations and vaccines made therefrom. Peptides derived from circumsporozoite protein of Plasmodium falciparum and/or the circumsporozoite protein of P. vivax, are covalently coupled, via spacer molecules to carrier proteins. P. falciparum and P. vivax vaccines, and P. falciparum-P. vivax bivalent vaccines, are disclosed.

In one embodiment, the present invention comprises a method of covalently coupling a peptide of the following amino acid composition, $(ASN:ALA:ASN:PRO)^n$, where n = 1-50, to a suitable carrier protein such as $CRM_{197}$, diphtheria toxoid, toxin A, choleragenoid, and meningococcal group B outer membrane proteins, using a spacer molecule such as succinic anhydride and/or adipic acid dihydrazide (hereinafter referred to as ADM), and a coupling agent such as 1-ethyl-3-dimethylaminopropyl) carbodiimide hydrochloride (hereinafter referred to as EDEC).

In another embodiment, the present invention comprises a method of reacting a peptide of the following amino acid composition, H-ASN:PRO:ASN:ALA: ASN:PRO:ASN:ALA:ASN:PRO:ASN:ALA-OH, (hereinafter referred to as Mpep), with ammonium chloride at a pH of 4-6, the product being hereinafter referred to as Mpep(N). The Mpep or Mpep(N) is covalently coupled to a carrier such as $CRM_{197}$, diphtheria toxoid, toxin A, choleragenoid, and meningococcal group B outer membrane proteins, using a spacer molecule, and a coupling agent.

In yet another embodiment, the present invention comprises a method for the covalent coupling of a peptide with the following amino acid composition, $MET:ASP:PRO[(ASN:ALA:ASN:PRO)_{15}$ -

(ASN:VAL:ASP:PRO)]$_2$LEU:ARG (hereinafter referred to as $R_{32}$-LeuARG), to a suitable carrier protein using a spacer molecule and a coupling agent.

In a further embodiment of the invention, the peptide (GLY:ASP:ARG:ALA:ASP:GLY:GLU:PRO:ALA)$^n$, hereinafter referred to as Vpep, where n = 1-50, is coupled to a suitable carrier protein, using a spacer molecule and a coupling agent.

In a further embodiment of the invention, there is disclosed a method of solubilizing and detoxifying gram negative outer membrane proteins with anahydride thus permitting use of the treated proteins as carrier proteins.

## DETAILED DESCRIPTION OF THE INVENTION

The present invention is directed to an immunizing preparation composed of peptides sharing antigenic determinants with the circumsporosoite (CSP) of P.falciparum or P. vivax. These novel immunizing agents are derived by synthesizing peptides with a known amino acid sequence which share homology to the CSP of P. falciparum or P. vivax. These non-immunogenic peptides are covently linked to carrier proteins using spacer molecules and coupling agents to render them immunogenic and suitable as vaccines for human use. These vaccines are useful for active immunization against malaria caused by the agent P. falciparum or P. vivax when administered by the parenteral, oral, or nasopharyngeal route.

The components of the conjugate vaccines can be prepared as follows.

The carrier protein selected should advantageously be nontoxic, nonpyrogenic, water soluble, pharmaceutically acceptable, of a size capable of producing a human immune response, capable of illiciting antibodies against a second infectious disease, stable at a pH of 2-12, hypoallergenic, and having relatively high number of reactive groups for coupling. Carrier proteins such as Diphtheria toxoid, $CRM_{197}$, Toxin A, Choleragenoid and gram negative outer membrane proteins such as Group B meningococcal outer membrane proteins can be used in the subject invention.

Diphtheria toxin can be purified from culture supernatants of Corynebacterium diphtheriae PW8 by ammonium sulfate precipitation, ion exchange chromatography, and gel filtration, Holmes, R.K., Infect. Immun. 12:1392 (1975). Detoxification can be accomplished by the addition of formalin and lysine to a toxin solution and incubation at 35°C for 1 month. The resulting toxoid passes all requirements set forth by the World Health Organization for diphtheria toxoid for human use.

$CRM_{197}$m a nontoxic protein which crossreacts immunologically with diphtheria toxin, can be purified from culture supernatants of C. diphtheriae C7 ($\beta^{tox-197}$) as described above Holmes, R.K. et al (1975) supra.

Toxin A can be purified from the culture supernatant of Pseudomonas aeruginosa PA103 by diafiltration ammonium sulfate precipitation and ion-exchange chromatography as described by Cryz, S.J. et al, Infect. Immun. 39:1072 (1983). Other immunologically related crossreacting materials may also be used

Choleragenoid can be isolated by treating purified cholera toxin under acidic conditions followed by gel filtration, Wong, K.H. et al, Biol. Stand. 5:197 (1977). Any choleragenoid-like molecule such as the B subunit of Escherichia coli heat-labile toxin can also be used.

Group B meningococcal outer membrane proteins can be purified from Neisseria meningitidis as follows. Killed bacteria is homogenized in a Tris-EDTA buffer. The supernatant is centrifuged at 100,000 x g for 2 hours, and the pellets collected. The outer membrane-containing pellets are suspended in 1% Empigen BB at pH 8, and incubated for 16 hours at 4°C. The solution is then sonicated. Ammonium sulfate is added to equal 500 g/L. The resulting precipitate is collected by centrifugation. The precipitate is dissolved in a Tris-NaCl EDTA, Empigen buffer and sonicated. The sonicate is extensively dialyzed against the above buffer. The solution is filtered through a 0.22 µM membrane filter. The solution is mixed with 3 volumes of cold ethanol and the precipitate collected. The pellet is suspended in a sodium phosphate, NaCl, Zwittergent buffer at pH 8. Other solubilized gram negative outer membrane proteins may also be used. Advantageously proteins from Neisseria, such as N. gonorrhoeae or other N. menigitidis can be used.

A peptide of the following amino acid composition, H-ASN:PRO:ASN:ALA:ASN:PRO:ASN:ALA:ASN:PRO:ASN:ALA-OH, i.e. Mpep, can be synthesized on a solid phase resin system and obtained from Bachem Feinchamikalen AG, Basel Switzerland.

The coupling agent used in advantageously a water soluble carbodiimide. An advantageous example of a coupling agent is 1-ethyl-3-(dimethylaminopropyl) carbodiimide (EDEC) having the formula:

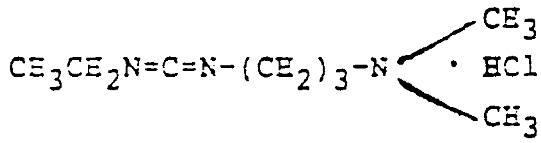

Advantageously the coupling agent generates a reative intermediate of the carrier protein permitting reaction between the carrier protein and the spacer molecule.

The spacer molecule used should advantageously be non-toxic, nonpyrogenic stable at pH 2-12, and having one or more reactive groups. The spacer molecule used is advantageously a dicarboxylic acid dihydrazide of the formula:

$NH_2$ -NH -CO -$(CH_2)_n$ -CO -NH -$NH_2$

where $n = 1$-20.

Advantageous examples of spacers are adipic acid dihydrazide (ADH), succinic anhydride, oxalic anhydride, maleic anhydride and phthalic anhydride, as well as other suitable anhydrides, or mono or dihydrazides which are composed of 1-20 carbon atoms. The use of these spacers permits a high ratio of peptide to protein carrier.

In the case of meningoccal outer membrane proteins, treatment with succinic anhydride or other suitable anhydrides renders the protein water soluble. It should be noted that the treatment with this spacer/solubilizing agent can be used on all gram negative outer membrane proteins including proteins from all Neisseria such as N. meningitidis and N. gonorrhoeae, to form soluble nontoxic carriers. The meningococcal proteins or other gram negative outer membrane proteins (OMP), are insoluble in the absence of detergent or other solubilizing agents such as polysaccharides or other moieties with some hydrophobic regions. The succinic anhydride treated OMP are soluble and the conjugates retain solubility. The conjugates are less pyrogenic in rabbits that the meningococcal proteins prior to treatment with succinic anhydride indicating that the treatment solubilizes as well as detoxifies these proteins thus permitting use as carriers.

Before conjugating the peptide to the carrier, the peptide can be pretreated with a compound such as ammonium chloride to block carboxy groups. Alternatively, the peptide can be treated with a compound such as succinic anhydride to block amino groups of the peptide. When a compound such as succinic anhydride is used in the manner, it also contributes to the spacer function.

Malaria conjugate vaccines described below were synthesized utilizing either succinic anhydride and/or adipic acid dihydrazide (ADH) as spacer molecules to facilitate the covalent coupling of peptides crossreacting immunologically with the CSP of P. falciparum or P. vivax to various carrier proteins.

The conjugation conditions employed as part of the present invention were effective at covalently linking malaria peptides or $R_{32}$-LeuARG to the various carrier proteins. The molar ratios of peptide to protein carrier ranged from 5:1 to 18:1, while the molar ratio of $R_{32}$-LeuARG to toxin A was 13:1.

As seen in the Western blots of Figures 1-4, crosslinking of conjugates occurs resulting in complexes of a variety of molecular weights.

The vaccines of the subject invention can be combined with a suitable adjuvant such as aluminium hydroxide or aluminium phosphate.

The following non-limiting examples illustrate the invention in more detail.

## Example 1

### $CRM_{197}$(S)-Mpep(N)

$CRM_{197}$ was entensively dialyzed against 0.1 M $NaPO_4$, pH 6.5. The material was transferred to a 125 ml erlenmeyer flask and the pH raised to 8.0.

Succinic anhydride was recrystallized by the addition of 70 ml of boiling acetic acid to 10 g of succinic anhydride. The solution was allowed to cool at 4°C and crystals were formed. The excess fluid was removed, the crystals were washed with anhydrous ether x 3, and the crystals were ground with a mortar and pestle and stored at -20°C under nitrogen.

Recrystallized succinic anhydride, 450 mg, was added to 30 mg of $CRM_{197}$ in 30 ml volume with constant stirring. The pH was maintained at 8.2 by the addition of NaOH. Two hours later, an additional 450

mg of succinic anhydride was added and the pH was maintained at 8.2 for two more hours. These reactions were performed at 25°C, the product being hereinafter referred to as $CRM_{197}$(S).

The reaction mixture was transferred to dialysis tubing and dialyzed against 2 L of 0.1 M $NaPO_4$ pH 6.5, buffer. Fluid was changed 4 times. The fluid was removed from the dialysis bag, filter sterilized through a 0.45 µM filter, and stored at 4°C. Volume was approximately 60 ml.

60 mg of Mpep was dissolved in 12 ml of 2 M ammonium chloride. The pH was 4.4. This procedure blocks the carboxy-terminus of the peptide, thus forming Mpep(N). Therefore, the subsequent steps described below will proceed through reactive groups expressed by the peptide, other than the carboxy terminus thereby potentially limiting antigenic alteration and self-polymerization of the peptide.

250 mg of 1 ethyl-3-(3-dimethylaminopropyl) carbodiimide hydrochloride (EDEC) was added to the peptide solution. The pH was lowered to 4.7 with 0.2 M HC1. The pH was monitored and kept at 4.7. Thirty minutes later, an additional 250 mg EDEC was added and the pH maintained at 4.7. Another 30 minutes later, an additional 250 mg of EDEC was added and the pH maintained at 4.7. After 3 additional hours, the pH was raised to 6.9 with NaOH and the mixture stored at 4°C. The material was shell frozen at -70°C and lyophilized.

The ammonium chloride treated peptide (Mpep(N)) was dissolved in 12 ml of sterile water and run over a Sephadex G10 column in two successive runs. This was to remove unreacted agents. Fractions containing peptide were identified by UV absorbance at OD 220, pooled, and lyophilized.

$CRM_{197}$(S) was covalently coupled to Mpep(N) in the following manner. 30 mg of succinic anhydride treated $CRM_{197}$($CRM_{197}$(S)) in 60 ml was mixed with 30 mg of ammonium chloride treated peptide in 10 ml water. The pH was lowered to 5.5 with 2 N HC1. 500 mg of EDEC was added and the mixture was allowed to stir gently for 5 hours. Then 500 mg of EDEC was added and stirred for 20 minutes. Again, 500 mg of EDEC was added and stirred for 20 minutes. Then an additional 500 mg of EDEC was added and stirred for 1 hour. The pH was 5.4 at this time. The reaction mixture was transferred to dialysis tubing and dialyzed against 0.1 M $NaPO_4$, pH 7.05. The solution was removed aseptically from the dislysis bag and allowed to pass through a 0.8 µM P.E. filter with gravity flow and then through a 0.45 µM filter. The solution was concentrated to 25 ml utilizing a sterile autoclaved Amicon concentration unit and a YM30 membrane. The solution was then passed through a 0.22 µM filter and stored at 4°C.

## Example 2

### Choleragenoid(S)-Mpep(N)

Choleragenoid was extensively dialyzed against 0.1 M $NaPO_4$, pH 6.5, at 4°C. 45 mg of dialyzed choleragenoid was placed in a 50 ml erlenmeyer flask and the pH was raised to 8.0. 200 mg of succinic anhydride was added and the pH maintained at 8.2 with 1 N NaOH. After 30 minutes, an additional 200 mg of succinic anhydride was added and the pH brought back to 8.2 with 1 N NaOH. 1 hour later, 200 mg of succinic anhydride was added and the pH maintained at 8.1 for the next hour. The solution was then transferred to a dialysis bag and dialyzed against 0.1 M $NaPO_4$, pH 6.5.

Mpep(N) was prepared in Example 1. 45 mg of choleragenoid(S) was placed in a 250 ml erlenmeyer flask with the desalted Mpep(N). The pH was lowered to 5.4 with 2 N HCl and 500 mg of EDEC was added. After 30 minutes of gentle stirring, an additional 500 mg of EDEC was added. Two more 500 mg additions of EDEC were made at 30 minute intervals and the solution was allowed to stir gently at 4°C for 5 hours. The reaction mixture was transferred to dialysis tubing and dialyzed against 0.1 M $NaPO_4$, pH 7.05. The material was removed from the dialysis bag, passed through a 0.22 µM filter, and stored at 4°C.

## Example 3

### Meningoccal 2B protein(S)-Mpep(N)

90 mg of meningococcal 2B protein in 37 ml was transferred to a 125 ml erlenmeyer flask. 450 mg of succinic anhydride was added and the pH titrated back to 9.2 with 1 N NaOH. Treatment of the

meninggococcal group B outer member proteins with succinic anhydride made the proteins water soluble. The protein solution became very clear at this point. The pH was maintained at 8.1 for 1 hour. Then 450 mg of succinic anhydride was added and the pH was maintained at 8.0 for 2 hours. The reaction mixture was gently stirred for 3 more hours during which time the pH dropped to 7.0. The reaction mixture was then dialyzed against 0.1 M $NaPO_4$, pH 7.05, buffer. The proteins remained soluble in the absence of detergent.

30 mg of Mpep(N) in 10 ml was added to 40 mg of succinic anhydride treated meningococcal 2B protein in an erlenmeyer flask. The pH was lowered to 5.5 with 2N HCl and 500 mg of EDEC was added and allowed to stir gently for 5 hours at 25°C. Three additions of 500 mg of EDEC were then made at 20 minute intervals, followed by 1 hour of stirring. The reaction mixture was then transferred to dialysis tubing and dialyzed against 0.1 M $NaPO_4$, pH 7.05. The material was removed from the dialysis bag, passed through a 0.22 μM filter, and stored at 4°C.

Example 4

Choleragenoid(ADH)-Mpep(S) (using adipic acid dihydrazide and succinic anhydride as spacer molecules)

Choleragenoid in 0.1 M $NaPO_4$, pH 7.0, (13 mg) in 5.4 ml was placed in a 25 ml flask. 300 mg of adipic acid dihydrazide (ADH) was added and the pH lowered to 5.2. 120 mg of EDEC was added and the pH was maintained at 5.5 with 0.1 N HCl. 30 minutes later, 120 mg of EDEC was added and the pH was maintained between 5.5-5.8. 1 hour later, 120 mg of EDEC was added, allowed to stir for 1 hour, and transferred to a dialysis bag. The reaction mixture was dialyzed against 0.1 M $NaPO_4$, pH 6.5. The resulting product is referred to as choleragenoid (ADH)>

20 mg of Mpep was suspended in 5 ml of 0.1 M $NaPO_4$ and the pH was raised to 8.2 with 1 N NaOH. 150 mg of succinic anhydride was added and the pH was maintained at 8.0 with 1 NNaOH. 90 minutes later, 150 mg of succinic anhydride was added and the pH maintained at 8.0 for the next 60 minutes. Gentle stirring proceeded for 2.5 hours at which time the pH was 7.0. The solution was run over a Sephadex G10 column and the appropriate fractions lyophilized. The resulting product is referred to as Mpep(S).

To 13 mg of choleragenoid (ADH) in 25 ml of 0.1 M $NaPO_4$, pH 6.5, was added 20 mg of Mpep(S). The pH was lowered to 4.5. 500 mg of EDEC was added and the pH lowered to 4.5. Three additional 500 mg amounts of EDEC were added at 20 minute intervals after which the solution was stirred for 20 minutes. The pH was adjusted to 6.0 and the mixture stirred for 5 hours at 4°C. The reaction mixture was transferred to a dialysis bag and dialyzed against 0.1 M $NaPO_4$, pH 7.05. The contents were then concentrated against a PM10 Amdcon filter to 20 ml and then filtered through a 0.22 μM filter.

Example 5

Toxin A (ADH)-$R_{32}$LeuARG(S)

80 mg of $R_{32}$-LeuARG was placed in a 50 ml flask, The pH was adjusted to 8.0. 225 mg of succinic anhydride was added and the pH was maintained at 8.0. 1 hour later, 180 mg of succinic anhydride was added, with pH titration, followed in 1 hour by another 180 mg addition of succinic anhydride. The reaction was allowed to stir gently for 5 hours. It was then dialyzed against 0.1M $NaPO_4$, pH 6.5. Upon removal from the dialysis bag, it was filtered through a 0.45 μM filter. This is termed $R_{32}$-LeuARG(S).

$R_{32}$-LeuARG(S) was placed in a 50 ml flask, the pH was lowered to 4.4, and 300 mg of EDEC was added. 32 mg of toxin A coupled to ADH (Toxin A (ADH)) was alowly added over a 30 minute period with gentle stirring. The solution became somewhat turbid once the toxin A(ADH) was added. 300 mg of EDEC was then added, followed by two more additions of 300 mg of EDEC at hourly intervals. 30 minutes after the last addition of EDEC, the pH was raised to 7.0, the reaction mixture was transferred to a dialysis bag, and dialyzed against 0.1 M NaPOH buffer, pH 7.01.

Example 6

Choleragenoid(S)-$R_{32}$LeuARG(N)

Choleragenoid was coupled to succinic anhydride as described above (Example 4). 90 mg of $R_{32}$-LeuARG in a volume of 23 ml was placed in a 125 ml sterile flask. 23 ml of 2 M ammonium chloride was added. The pH of the solution was lowered to 4.5 with HCl. 500 mg of EDEC was added and the pH adjusted to 4.5. 90 minutes later, 500 mg of EDEC was added and the pH lowered to 4.5. 60 minutes later, 500 mg of EDEC was added and the pH was allowed to remain at 4.6. One hour after the last addition of EDEC, the contents were transferred to a dialysis bag and dialyzed against 0.1 M $NaPO_4$, pH 6.5.

30 ml of choleragenoid(S) (approximately 25 mg) was mixed with 31 ml of $R_{32}$-LeuARG(N). The pH was lowered to 5.45 with 0.5 ml 2 N HCl. 500 mg of EDEC was added every 30 minutes for 90 minutes. One hour after the last addition of EDEC, the contents were dialyzed against 0.1 M $NaPO_4$, pH 7.05. The contents were then dialyzed against 0.1 M $NaPO_4$, pH 7.0, using dialysis tubing with a 50,000 molecular weight cutoff. The material was passed through a 0.22 $\mu$M filter.

Example 7

Meninogococcal Outermembrane Protein(S)-Mpep

Meningoccal outer member proteins (OMP) were treated with succinic anhydride as described in Example 3. The product is referred to as Meningococcal 2B(S)>

27 ml of Meningoccal 2B(S) containing 50 mg of protein was mixed with 25 mg of M-pep previously dissolved in 3 ml $H_2O$. The pH of the mixture was lowered to 6.2. 100 mg of EDEC was added and the mixture was allowed to stir for 4 hours. Two hundred mg of EDEC was then added. After 30 minutes, 25 mg Mpep was added plus 200 mg EDEC. 30 minutes later 200 mg of EDEC was added. Three hours later 200 mg EDEC was added. The mixture was allowed to stir for 3 hours and then dialyzed with 3 changes against 5 liters of PBS>

Example 8

Meningococcal OMP(S)-V-pep

45 mg of Meningococcal OMP was treated with succinic anhydride as described in Example 3 had the pH carefully lowered to 6.10. 200 mg of EDEC was then added. 50 mg of Vpep dissolved in $H_2O$ was added drop by drop over a 15 minute period. The pH was lowered to 5.5. After one-half hour 100 mg of EDEC was added. The solution became somewhat opalescent. 3 hours after reaction began the solution was dialyzed against 5 liters of PBS.

Example 9

Toxin A (ADH)-$R_{32}$LeuARG

10 mg of toxin A (ADH) was prepared as described in Example 5 in 2.7 ml mixed with 50 mg of $R_{32}$ LeuARG. The pH was lowered to 4.80 with 1 M HCl and 100 mg of EDEC was added. After 20 minutes of constant stirring, 100 mg of EDEC was added and the pH was lowered to 4.8. After 60, 100 ml 140 minutes

from the onset of the reaction, 100 mg of EDEC was added. Constant stirring was maintained at 25°C for 7 hours after initiation of the reaction and then dialyzed against 3 changes of 5 liters of PBS. The solution was then ultrafiltered with an Amicon PM 30 membrane 30 volumes to remove noncovalently bound $R_{32}$ LeuARG and redialyzed against PBS. Final vaccine was filtered with a 0.22μ filter.

Example 10

Toxin A (ADH)-Mpep and Vpep

50 mg of Toxin A (ADH) in 138 ml was prepared as described in Example 5 and the pH was lowered to 4.830. 50 mg of Mpep was dissolved in 3 ml $H_2O$. 250 mg of EDEC was added. 500 μl of peptide was added drop by drop over 10 minutes. 100 mg EDEC was added after the pH was lowered to 4.700. 1.5 ml of peptide was added drop by drop over 20 minutes. 100 mg EDEC was added following by the remaining 500 μl of peptide. After 5 hours total reaction time the solution was dialyzed against four changes of 5 liters of PBS.

50 mg of Toxin A (ADH) in 13.8 ml was prepared and the pH lowered to 4.8 as above. 200 mg EDEC was added. 50 mg of Vpep(n = 2) was dissolved in 3 ml $H_2O$. 2 ml of peptide was added dropwise over 20 minutes. 100 mg EDEC was added and the remaining ml added over the next 10 minutes. Forty minutes and sixty minutes from the beginning of the reaction, 100 mg EDEC was added. Three hours after the beginning of the reaction the material was dialyzed against 4 changes of 5 liters of PBS.

Features of the present invention will become fully apparent when the following detailed description of a preferred embodiment of the invention is read in conjunction with the accompanying tables; wherein:

Table A shows the composition of several vaccines which have been prepared.

Table A

Vaccine Composition

| Conjugate Vaccine | Peptide | (Treatment) | Carrier Protein | (Treatment) |
|---|---|---|---|---|
| a) $R_{32}$LeuARG(S) Toxin A(ADH) | $R_{32}$LeuARG | Succinic Anhydride | Toxin A | Adipic Acid Dihydrazide |
| b) $R_{32}$LeuARG(N) Choleragenoid(S) | | $R_{32}$LeuARG Chloride | Ammonium | CholeragenoidSuccinic Anhydride |
| c) Mpep D.T.(ADH) | Mpep (Falcip) | - (i.e, none) | Diptheria Toxid (DT) | Adipic Acid Dihydrazide |
| d) Mpep(N)Meningococcal 2B(S) | Mpep | Ammonium Chloride | Meningcoccal Outermembrane | Succinic Anhydride |
| e) Mpep(N) $CRM_{197}$(S) | Mpep | Ammonium Chloride | $CRM_{197}$ | Succinic Anhydride |
| f) Mpep(N) Choleragenoid(S) | Mpep | Ammonium Chloride | Choleragenoid | Succinic Anhydride |
| g) Mpep(S) Choleragenoid(ADH) | Mpep | Succinic Anhydride | Choleragenoid | Adipic Acid Dihydrazide |
| h) $R_{32}$LeuARG Toxin A(ADH) | $R_{32}$LeuARG | - | Toxin A | Adipic Acid Dihydrazide |
| i) Mpep Meningococcal 2B(S) | Mpep | - | Meningococcal OMP | Succinic Anhydride |
| j) MPep $CRM_{197}$(S) | Mpep | - | $CRM_{197}$ | Succinic Anhydride |
| k) Mpep DT(ADH) | Mpep | - | Diptheria Toxoid | Adipic Acid Dihydrazide |
| l) Mpep Tetanus(ADH) | Mpep | - | Tetanus Toxoid | Adipic Acid Dihydrazide |
| m) Mpep Cholergenoid(ADH) | Mpep | - | Choleragenoid | Adipic Acid Dihydrazide |
| n) Vpep Meningococcal2B(S) | Vivax (n=2 repeat units) | - | Meningococcal 2B(Modified) | Succinic Anhydride |



BAD ORIGINAL

| | | | | |
|---|---|---|---|---|
| o) Vpep Toxin A(ADH) | Vivax (n=2) | - | Toxin A | Adipic Acid Dihydrazide |
| p) Vpep Choleragenoid(ADH) | Vivax (n=2) | - | Choleragenoid | Adipic Acid Dihydrazide |
| q) Vpep(S) Choleragenoid(ADH) | Vivax (n=2) | Succinic Anhydride | Choleragenoid | Adipic Acid Dihydrazide |
| r) Vpep $CRM_{197}$(S) | Vivax (n=2) | - | $CRM_{197}$ | Succinic Anhydride |
| s) Vpep DT(ADH) | Vivax (n=2) | - | Diptheria Toxoid | Adipic Acid Dihydrazide |

Table 1 shows the chemical composition of conjugate vaccines as molar ratios of malaria peptide per carrier protein in one embodiment of the present invention.

Table 1.

Composition of Selected Malaria Conjugate Vaccines

| Conjugate | Molar ratio[1] (peptide/carrier protein) |
|---|---|
| Toxin A(ADH)-$R_{32}$-LeuARG(S) | 13:1 |
| Choleragenoid(S)-Mpep(N) | 12.5:1 |
| Choleragenoid(ADH)-Mpep(S) | 5:1 |
| $CRM_{197}$(S)-Mpep(N) | 18:1 |
| Meningococcal B(S)-Mpep(N) | 6:1 |

[1] Determined by amino acid analysis based upon aspartic acid content.

Table 2 shows the biological characteristics of the conjugate vaccines in relation to their safety when used as human vaccines as a second embodiment of the present invention.



BAD ORIGINAL

Table 2.
Biological Characteristics of Malaria Peptide Conjugate Vaccines

| Conjugate[1] | Pyrogenicity[2] (°C) | General Safety[3] | Sterility[4] |
|---|---|---|---|
| $R_{32}$-LeuARG (polymerized) | 0 | nontoxic | sterile |
| Toxin A(ADH)-$R_{32}$-LeuARG(S) | 0.2 | nontoxic | sterile |
| Choleragenoid(S)-$R_{32}$-LeuARG (N) | 0 | nontoxic | sterile |
| Diphtheria toxoid(ADH)-Mpep | 0.2 | nontoxic | sterile |
| Meningococcal 2B(S)-Mpep(N) | 0.3 | nontoxic | sterile |
| $CRM_{197}$(S)-Mpep(N) | 0.3 | nontoxic | sterile |
| Choleragenoid(S)-Mpep(N) | 0 | nontoxic | sterile |
| Choleragenoid(ADH)-Mpep(S) | 0.4 | nontoxic | sterile |

1 Conjugates (prior to absorption onto $Al(OH)_3$) were dissolved in saline to a final concentration of 10 μg/ml except for $R_{32}$-LeuARG-choleragenoid conjugate, which was at 2 μg/ml. Each rabbit received 1 ml of material per kg bodyweight by the intravenous route.

2 Maximum increase in body temperature noted for 4 hours post-injection of 10μ/kg body weight. Choleragenoid (ADH)-Mpep(S) gave no temperature rise at 2μ/kg body weight. Conjugates i) - p) and s) of Table A all passed (i.e. <.2) at 50μ/kg.

3 Mice (18-20 g) and guinea pigs (250-300 g) each received 100 μg of material intraperitoneally. There were no deaths and all animals gained weight. Determined according to article V.2.1.5 of the European Pharmacopoeia.

4 Determined according to article V.2.1.1 of the European Pharmacopoeia.

Table 3 shows the magnitude of the immune response in rabbits to the malaria peptide moiety of the conjugate vaccines.



BAD ORIGINAL

Table 3.
Immune Response in Rabbits Following Immunization with Conjugate Vaccines

| Immunogen[1] | Mean anti-malaria CSP response (range)[2] | |
|---|---|---|
| $R_{32}$-LeuARG (polymerized) | 48 | (9-54) |
| Toxin A(ADH)$R_{32}$-LeuARG(S) | 8107 | (5712-15,017) |
| Choleragenoid (S)-$R_{32}$-LeuARG(N) | 529 | (53.4-851) |
| Diphtheria Toxoid(ADH)-Mpep | 3126 | (70-5808) |
| Meningococcal B(S)-Mpep(N) | 3005 | (489-5225) |
| $CRM_{197}$(S)-Mpep(N) | 1674 | (566-3147) |
| Choleragenoid(S)-Mpep(N) | 988 | (197-2339) |
| Choleragenoid(ADH)-Mpep(S) | 1452 | (621-2356) |

[1] Rabbits (groups of 3) were immunized on days 0 and 28 with 100 µg of vaccine in 4% (wt/vol) $Al(OH)_3$. Sera was obtained on day 42. Coupling agent used was EDEC in all cases.

[2] ELISA antibody titers are expressed as units (optical density x serum dilution/ml). Antibody response was detected using a peptide antigen which crossreacts with the circumsporozoite protein (CSP).

Table 4 shows the magnitude of the immune response in rabbits to the carrier protein moiety of the conjugate vaccines.



BAD ORIGINAL

Table 4.
Immunoglobulin G Antibody Response in Rabbits to Carrier Proteins Following Vaccination with Malaria Peptide Conjugates

| Immunogen[1] | Mean anti-carrier protein IgG ELISA titer (range)[2] | |
|---|---|---|
| Normal Rabbit Serum | 8.3 | (1.2-44) |
| Toxin A(ADH)-$R_{32}$-LeuARG(S) | 126.6 | (79-208) |
| Choleragenoid(S)-$R_{32}$-LeuARG(N) | 10 | (2.5-24.5) |
| Diphtheria Toxoid(ADH)-Mpep | 157.3 | (45-322) |
| Meningococcal B(S)-Mpep(N) | 820 | (620-1200) |
| $CRM_{197}$(S)-Mpep(N) | 205.3 | (97-340) |
| Choleragenoid(S)-Mpep(N) | 25.6 | (2.5-30) |
| Choleragenoid(ADH)-Mpep(S) | 18,400 | (6450-36,000) |

1 Rabbits (groups of 3) were immunized on days 0 and 28 with 100 µg of vaccine in 0.4% (wt/vol) $Al(OH)_3$. Sera were obtained on day 42. Coupling agent used was EDEC in all cases.

2 ELISA antibody titers are expressed as units (optical density x serum dilution/ml). Antibody response is shown for the homologous protein carrier.

Table 5 is a second set of data for the vaccines and methods of Tables 3 and 4.



BAD ORIGINAL

Table 5.
Immune Response in Rabbits to Peptide Moiety and to Protein Carrier Following Vaccination With Malaria Peptide Conjugates

| Vaccine | Peptide ELISA* | Fold Rise** | Florescence*** | Protein ELISA* | Carrier Fold Rise** |
|---|---|---|---|---|---|
| $R_{32}$LeuARG(Polymerized) | 80 | 1.25 | 0 | | |
| Toxin A(ADH)-$R_{32}$LeuARG(S) | 6819 | 13.7 | 400 | 115 | 41.4 |
| Choleragenoid(S)-$R_{32}$LeuARG(N) | 674 | 10.5 | 400 | 5.6 | 0.97 |
| DT(ADH)-Mpep | 3019 | 14.8 | 400 | 115 | 21.3 |
| Meningococcal 2B(S)-Mpep (N) | 2950 | 36.9 | 400 | 780 | 28. |
| $CRM_{197}$(S)-Mpep (N) | 2000 | 44.6 | 100 | 180 | 91.5 |
| Choleragenoid(S) Mpep(N) | 1192 | 11.97 | 100 | 11.2 | 3.9 |
| Choleragenoid(ADH) Mpep(S) | 1708 | 26.6 | 400 | 14,358 | 3572 |

* Titers represent geometric means.

** Fold rises represent geometric mean fold rise.

*** Florescent Assay

Sporozoites from Falciparum malaria are fixed to a slide. Dilutions of rabbit antibody are incubated with the sporozites. The slide is washed and a florescenated goat anti rabbit antibody is incubated with the sporozoites. After washing the slides, they are read with a flucrescent microscope for the highest dilution of sera giving a flucrescent reaction.

Table 6 shows the results of simultaneously administering Mpep coupled to two different carrier proteins.

BAD ORIGINAL

Table 6

Anti-Mpep ELISA Titer following Immunization of Rabbits with a Bivalent Vaccine

| Antigen[1] | Anti-Mpep ELISA titer (range) |
| --- | --- |
| Mpep-ADH-CHOL | 3292 (2034 - 4955) |
| Mpep-ADH-CHOL + Mpep-ADH-Tetanus-toxoid | 11,066 (6,300 - 19,000) |
| Mpep-ADH-Tetanus-toxoid | 5012 (3034 -8095) |

[1] Rabbits (3) were immunized with 100/µg of vaccine intramuscularly on day 0 and 14. Serum was taken on day 28. For the combined vaccine group 50 µg of vaccine from each component was given at two different sites.

Table 7 shows the antibody response in humans to the synthetic peptide coupled to a carrier protein.



BAD ORIGINAL

Table 7

Antibody Response in Humans Following Vaccination with Malaria Peptide Conjugated with an Adjuvant Suitable for Human Use $Al(OH)_3$

| Vaccine[1] | Volunteer Nr. | Optical Density Units (ELISA) Week | | | Nr. volunteer mounting a response (≥12.5 OD units) |
|---|---|---|---|---|---|
| | | 0 | 4 | 10 | |
| Mpep-MENING B | 1 | 9 | 12 | 10 | |
| | 2 | 7 | 17 | 18 | |
| | 3 | 2.5 | 3 | 25 | 2/5 |
| | 4 | 5 | 17 | 19 | |
| | 5 | 6.5 | 7 | 6.5 | |
| Mpep-ADH-CHOL | 1 | 11.5 | 19 | 24 | |
| | 2 | 6 | 7.5 | 8 | 1/4 |
| | 3 | 8 | 8.5 | 8 | |
| | 4 | 6 | 6 | 7 | |
| [illegible] | 1 | 6 | 11 | 13 | |
| | 2 | 7 | 7.5 | 7 | 1/3 |
| | 3 | 2 | 3 | 7 | |
| Mpep-ADH-Suc-CHOL | 1 | 7.5 | 17 | 10.5 | |
| | 2 | 6 | 15 | 17 | 2/3 |
| | 3 | 6 | 6.5 | 7.5 | |



BAD ORIGINAL

[1] Vaccine 1: Meningococcal outer membrane protein was used as a carrier (Mpep - MENING-B).

Vaccine 2: Choleragenoid was used as a carrier coupled to NANP via ADH (Mpep - ADH - CHOL).

Vaccine 3: CRM 197 (a genetically derived diphtheria toxoid) was used as a carrier (Mpep - $CRM_{197}$).

Vaccine 4: Choleragenoid was used as a carrier protein and coupled to Mpep by use of both ADH and succinic anhydride (Mpep - ADH - Suc - CHOL).

The conjugates described above were designed to be suitable for human usage. Therefore, the malaria antigen, carrier protein, spacer molecule(s), and reaction conditions were selected to optimize the generation of an acceptable final product. However, while the above reagents themselves were neither toxic nor pyrogenic, it was a distinct possibility that once covalently coupled, one or more combinations could prove to be toxic and/or pyrogenic due either an increase in molecular weight or the generation of new antigenic determinants or reactive groups. Therefore, the toxicity and pyrogenicity of each conjugate vaccine was tested. All conjugates were nonpyrogenic, evoking an increase in body temperature of less than 0.5°C at doses of ≥2 µg/kg. This dose level exceeds the recommendation for other vaccines for human use such an Meningococcal A and C (recommended dose level of 0.05 µg/kg). The conjugate vaccines were nontoxic when 100 µg were administered intraperitoneally to either mice or guinea pigs. These tests were performed according to international regulatory guidelines (European Pharmacopoeia). These findings suggest that these conjugate vaccines would be well tolerated upon parenteral administration to humans.

Subsequently, the immune response elicited in rabbits following immunization with the above malaria conjugate vaccines was evaluated. First, the antibody response to the malaria antigen component of the vaccine was studies. Native (i.e., unconjugated) $R_{32}$-LeuARG was found to be non-immunogenic (48 mean ELISA units/ml of serum). However, conjugates comprised of $R_{32}$-LeuARG and either toxin A or choleragenoid was highly immunogenic (8107 and 3126 mean ELISA units/ml of serum, respectively). Similarly, immunization of rabbits with the peptide, ASN:PRO:ASN:ALA:ASN:PRO:ASN:ALA:ASN:PRO:ASN:ALA, in unconjugated form evoked no antibody response. Conjugates produced by covalently coupling said peptide to either diphtheria toxoid, toxin A, meningococcal group B outer membrane proteins, $CRM_{197}$, or choleragenoid were able to induce antibody to the carrier protein. However, when choleragenoid was coupled to either $R_{32}$-LeuARG or the above peptide via succinic anhydride, there was an extremely weak anti-carrier response. This would indicate that the procedure used to construct these 2 conjugates destroyed the vast majority of immunodominant antigenic determinants expressed by choleragenoid. In spite of this, these 2 conjugates were capable of eliciting a good anti-malaria peptide response.

To demonstrate that the above conjugates had potential for use as active vaccines against malaria, a limited trial was undertaken to evaluate the safety and immunogenicity of several conjugates in human volunteers. Volunteers were immunized with the following conjugates:

$CRM_{197}$-Mpep
choleragenoid-Mpep
choleragenoid-Mpep(ADH)
meningococcal B-Mpep

The vaccines were adsorbed onto $Al(OH)_3$, which functions as an adjuvant, and each volunteer received the equivalent of 100 µg of carrier protein intramuscularly in 0.5 ml. Reactions following vaccination were mild and transient, consisting primarily of local pain. To document a lack of systemic toxicity, the following analytical procedures were performed at the time of immunization and 1 week post-immunization; hemoglobin, hematocrit, erythrocyte count, white blood cell count, differential blood count, serum glutamate-oxalacetatetransamanase, serum glutamate-pyruvate-transamanase, alkaline phosphatase, creatinine, and bilirubin. Vaccination did not alter any of these paramenters, indicating a lack of toxicity.

While preferred embodiments of the invention have been described herein, it will be evident to those skilled in the art from a reading of the foregoing disclosure that in lieu of the peptide forming an antigenic determinant of circumsporozoite protein, a peptide forming an antigenic determinant of a) bacterial pili



BAD ORIGINAL

(attachment organelles) from organisms such as N. gonorrhoeae or P. aeruginosa, b) hepatitis B virus, c) HTLV III/LAV virus (e.g. gp 120), d) internal image antiidiotype antibody, can be used to form a conjugate. Various changes and modifications, especially pertaining to the spacer molecules used, number of amino acid repeat units in the peptide moiety, and modification of said peptides to block reactive groups prior to covalent coupling to carrier proteins, may be made without departing from the spirit of the present invention.

## Claims

1. A method for producing an immunogenic conjugate comprising:
covalently linking (i) a carrier protein to (ii) a peptide forming an antigenic determinant of circumsporozoite protein via (iii) at least one spacer molecule.

2. The method of claim 1 wherein said linking step comprises covalently binding said at least one spacer molecule to one of said carrier protein and said peptide to form complex A; and then covalently binding said complex A with the remaining one of said carrier protein and said peptide.

3. The method of claim wherein said linking step comprises covalently binding a first spacer molecule to said carrier protein to form complex A, and covalently binding a second spacer molecule to said peptide to form complex B, and then covalently linking complex A to complex B to form said conjugate.

4. The method of claim 1 wherein said peptide comprises the following amino acid sequence: $(ASN:ALA:ASN:PRO)^n$ where n = 1-50.

5. The method of claim 1 wherein said peptide comprises the following amino acid sequence: $(GLY:ASP:ARG:ALA:ASP:GLY:GLU:PRO:ALA)^n$ where n = 1-50.

6. The method of claim 1 wherein said peptide comprises the following amino acid sequence: $MET:ASP:PRO:[(ASN:ALA:ASN:PRO)_{15}ASN:VAL: ASP:PRO]_2$.

7. The method of claim 1 wherein said peptide comprises the following amino acid sequence: $MET:ASP:PRO[(ASN:ALA:ASN:PRO)_{15}ASN:VAL:ASP:PRO]_2LEU: ARG$.

8. The method of claim 1 wherein in said linking step additional peptide forming an antigenic determinant of circumsporozoite protein is linked directly to said carrier protein.

9. The method of claim 1 wherein before said linking step, said peptide is pretreated with an agent which selectively blocks amino groups or carboxylic groups.

10. The method of claim 9 wherein said peptide is pretreated with ammonium chloride.

11. The method of claim 9 wherein said peptide is pretreated with succinic anhydride.

12. The method of claim 1 wherein said carrier protein is selected from the group consisting of diphtheria toxoid, $CRM_{197}$, toxin A, choleragenoid, the B subunit of Escherichia coli heat-labile toxin, and meningococcal group B outer membrane proteins.

13. The method of claim 1 wherein said carrier protein is a gram negative outer membrane protein.

14. The method of claim 1 wherein said carrier protein is à Neisseria meningitidis outer membrane protein.

15. The method of claim 1 wherein said at least one spacer molecule is a dicarboxylic acid dihydrazide of the following composition, $NH_2-NH-CO-(CH_2)_n-CO-NH-NH_2$, where n = 1-20.

16. The method of claim 1 wherein said at least one spacer molecule is selected from the group consisting of succinic anhydride, adipic acid dihydrazind, oxalic anhydride, phthalic anhydride, and maleic anhydride, adipic acid dihydrazide, or mixtures thereof.

17. The method as in claim 1, wherein said linking step is performed using a water-soluble carbodiimide as a coupling agent.

18. The method of claim 17 wherein said water soluble carbodiimide is 1 ethyl-3-(dimethylaminopropyl)-carbodiimide.

19. An immunogenic conjugate capable of inducing a protective response in a mammal against malarial infection, said conjugate comprising:
(i) a carrier protein covalently bound to (ii) a peptide comprising at least one antigenic determinant of circumsporozoite protein, via (iii) at least one spacer molecule.

20. A conjugate as in claim 19 wherein said peptide comprises the following amino acid sequence: $(ASN:ALA:ASN:PRO)^n$ where n = 1-50.

21. A conjugate as in claim 19 wherein said peptide comprises the following amino acid sequence: $(GLY:ASP:ARG:ALA:ASP:GLY:ALU:PRO:ALA)^n$ where n = 1-50.

22. A conjugate as in claim 19 wherein said peptide comprises the following amino acid sequence: $MET:ASP:ALA[(ASN:ALA:ASN:PRO)_{15}ASN: VAL:ASP:PRO]_2$.

23. A conjugate as in claim 19 wherein said peptide comprises the following amino acid sequence: MET:ASP:PRO[(ASN:ALA:ASN:PRO)$_{15}$ASN:VAL:ASP:PRO]$_2$ LEU:ARG.

24. A conjugate as in claim 19 wherein said carrier protein is selected from the group consisting of diphtheria toxoid, $CRM_{197}$, toxin A, choleragenoid, the B subunit of Escherichia coli heat-labile toxin, and meningococcal group B outer membrane proteins.

25. A conjugate as in claim 19 wherein said carrier protein is a gram negative outer membrane protein.

26. A conjugate as in claim 19 wherein said carrier protein is a Neisseria meningitidis outer membrane protein.

27. A conjugate as in claim 19 wherein said at least one spacer molecule is a dicarboxylic acid dihydrazide of the following composition, $NH_2$-NH-CO-$(CH_2)_n$-CO-NH-$NH_2$ where n = 1-20.

28. A conjugate as in claim 19 wherein said at least one spacer molecule is selected from the group consisting of succinic anhydride, oxalic anhydride, phthalic anhydride, and maleic anhydride, adipic acid dihydrazide, or mixtures thereof.

29. A conjugate as in claim 19 wherein additional peptide forming an antigenic determinant of circumsporozoite protein is linked directly to said carrier protein.

30. A conjugate as in claim 19 further comprising an adjuvant.

31. A conjugate as in claim 30 wherein the adjuvant is selected from the group consisting of aluminum hydroxide and aluminum phosphate.

32. A method of producing immunity to malaria in a warm-blooded animal comprising administering the conjugate of claim 19 to the warm-blooded animal.

33. A method of forming a water soluble nontoxic carrier protein for use in a conjugate vaccine comprising:

treating a gram negative outer membrane protein with an anhydride.

34. The method of claim 33 wherein said anhydride is selected from the group consisting of succinic anhydride, maleic anhydride, oxalic anhydride and phthalic anhydride.

35. The method of claim 33 wherein said gram negative outer membrane protein is a Neisseria protein.

36. A method of producing immunity to malaria in a warm-blooded animal comprising administering at least two conjugates as claimed in claim 19 to said warm-blooded animal, wherein said carrier protein in each of said conjugates is different.

WESTERN BLOT OF MALARIA CONJUGATE VACCINE AND CARRIER PROTEINS DEVELOPED WITH MONOCLONAL ANTIBODY AGAINST MALARIA PEPTIDE

FIG.1

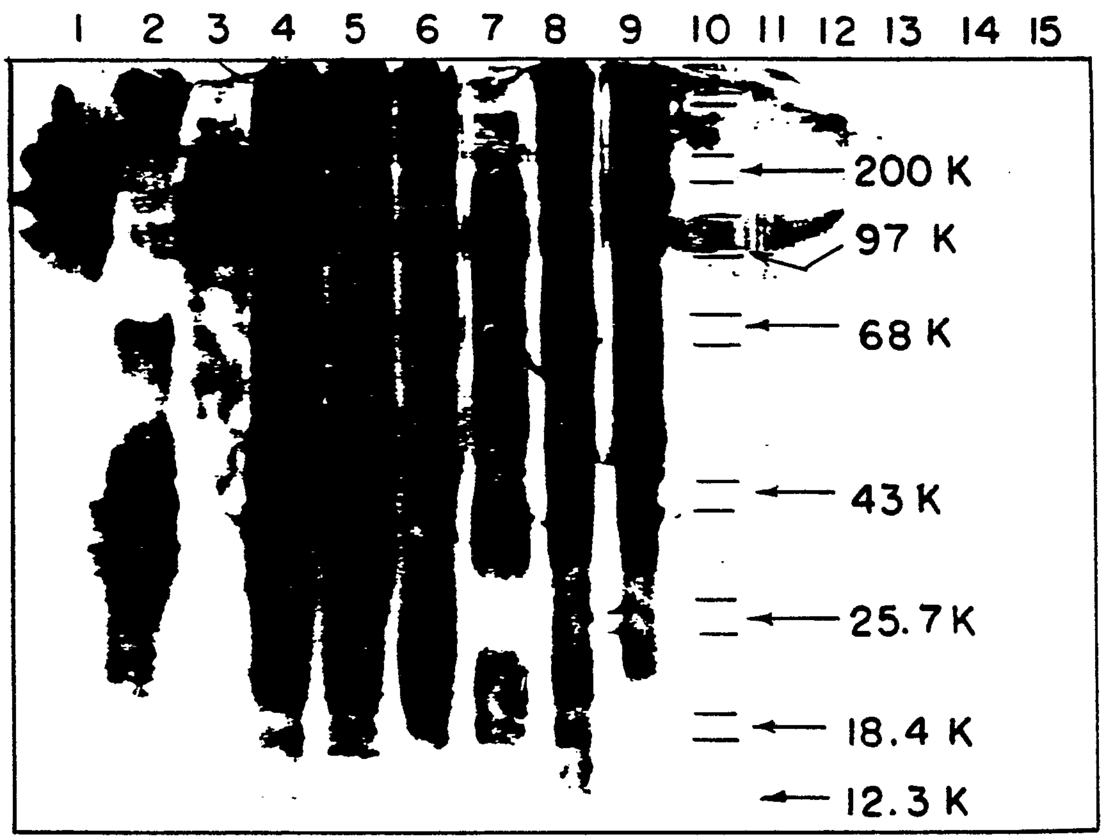


Protocol for Gels

1) $R_{32}$ LeuARG
2) $R_{32}$ leuARG (N)
3) $R_{32}$ LeuARG (S)
4) $R_{32}$ leuARG (S) - Toxin A (ADH)
5) $R_{32}$ leuARG (N)N - Choleragenoid (S)
6) Mpep (N) - Choleragenoid (S)
7) Mpep (S) - Choleragenoid (ADH)
8) Mpep (N - Meningococcal B(S)
9) Mpep(N) - $CRM_{197}$(S)
10) M.W. Standard
11) Toxin A (ADH)
12) Choleragenoid (S)
13) Choleragenoid (ADH)
14) Meningococcal B(S)
15) $CRM_{197}$(S)

VACCINE vs MIX OF MONOCLONAL ANTIBODIES
AGAINST CARRIER PROTEINS

FIG.2

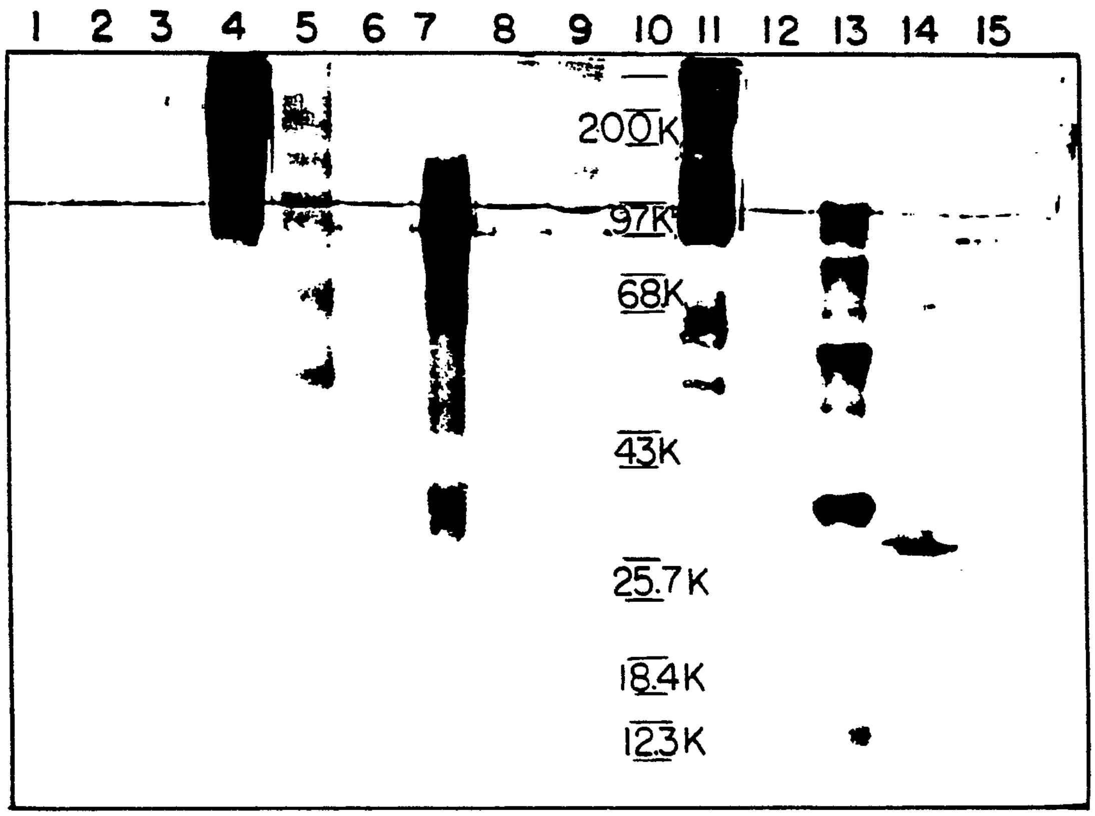


Protocol for Gels

1) $R_{32}$ LeuARG
2) $R_{32}$ leuARG (N)
3) $R_{32}$ LeuARG (S)
4) $R_{32}$ leuARG (S) - Toxin A (ADH)
5) $R_{32}$ leuARG (N)N - Choleragenoid (S)
6) Mpep (N) - Choleragenoid (S)
7) Mpep (S) - Choleragenoid (ADH)
8) Mpep (N - Meningococcal B(S)
9) Mpep(N) - $CRM_{197}$(S)
10) M.W. Standard
11) Toxin A (ADH)
12) Choleragenoid (S)
13) Choleragenoid (ADH)
14) Meningococcal B(S)
15) $CRM_{197}$(S)

FALCIPARUM AND VIVAX CONJUGATE VACCINE DEVELOPED WITH MONOCLONAL ANTIBODY AGAINST VIVAX AND FALCIPARUM PEPTIDE

FIG.3

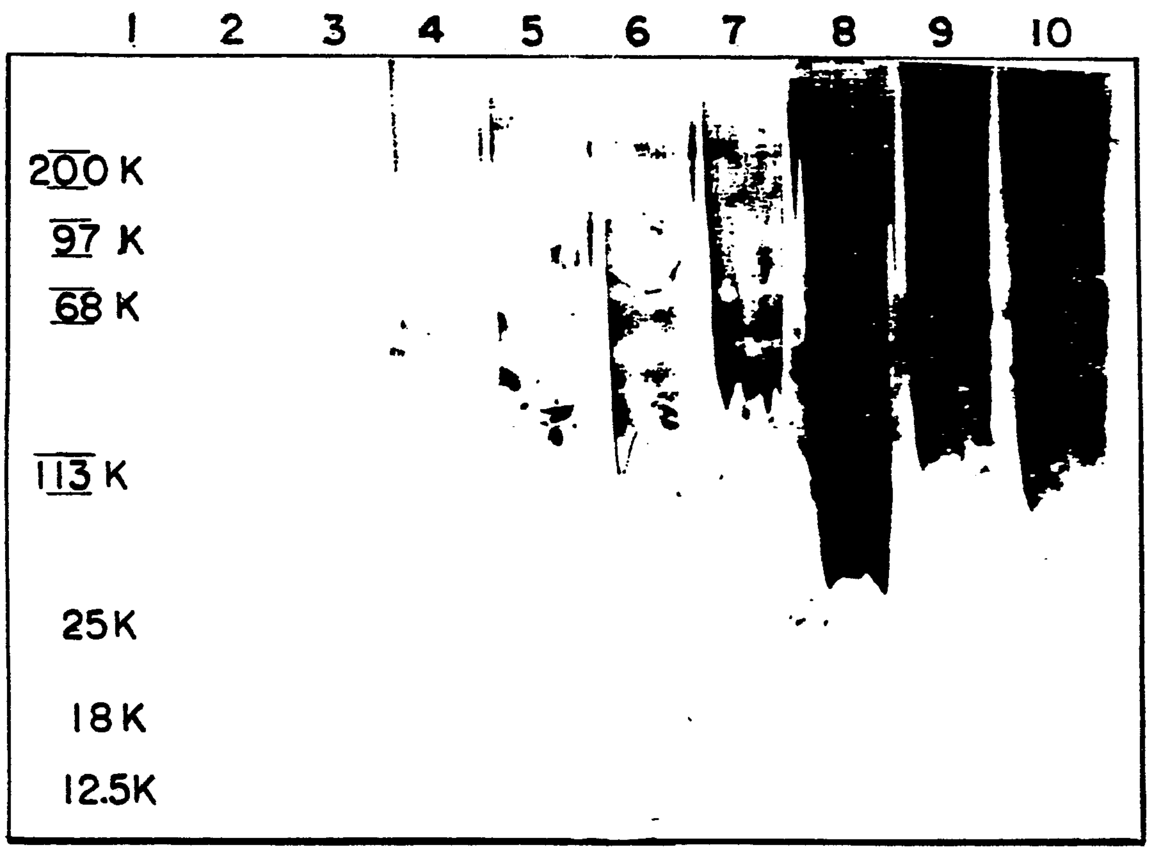


1) Meningococcal 2B(S)
2) $CRM_{197}$(S)
3) Choleragenoid (ADH)
4) Vpep (V=2) Meningococcal 2B(S)
5) Vpep (V=2) $CRM_{197}$(S)
6) Vpep (V=2) Choleragenoid (ADH)
7) Vpep (V=2) Choleragenoid (ADH)
8) Mpep Choleragenoid (S)
9) Mpep Meningococcal 2B (S)
10) Mpep $CRM_{197}$(S)

WESTERN BLOT OF VIVAX AND FALCIPARUM CONJUGATE VACCINES DEVELOPED WITH ANTIBODY AGAINST CARRIER PROTEIN

FIG.4

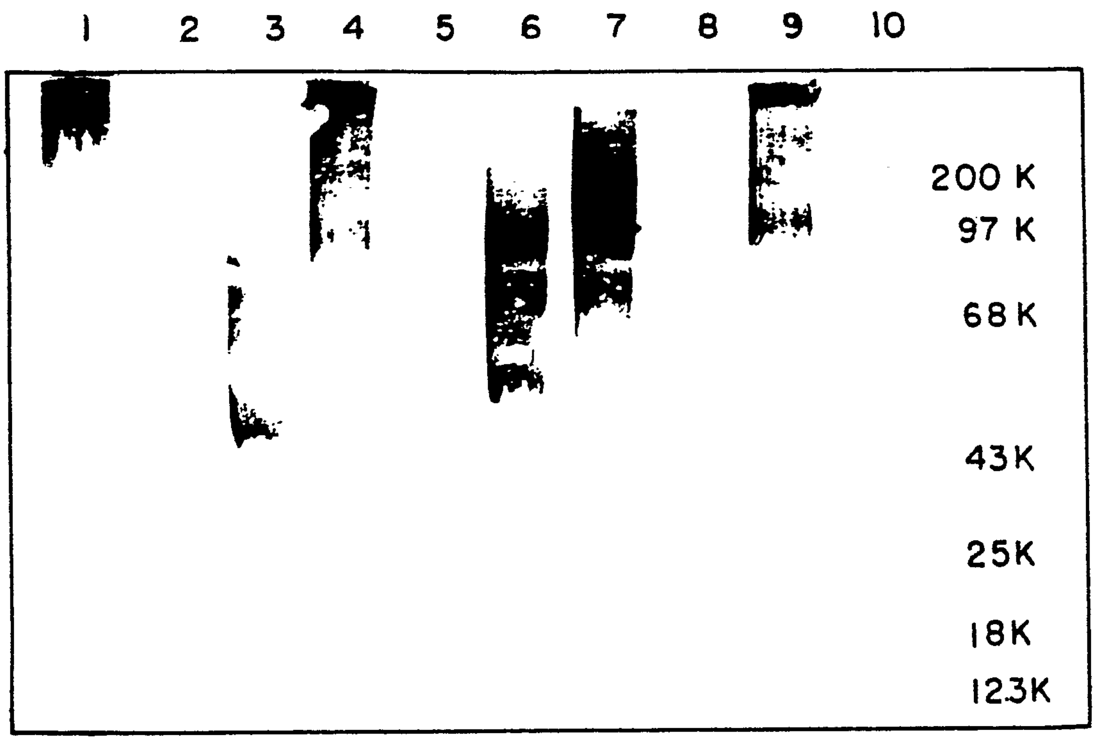


1) Meningococcal 2B(S)
2) $CRM_{197}$(S)
3) Choleragenoid (ADH)
4) Vpep (V=2) Meningococcal 2B(S)
5) Vpep (V=2) $CRM_{197}$(S)
6) Vpep (V=2) Choleragenoid (ADH)
7) Vpep (V=2) Choleragenoid (ADH)
8) Mpep Choleragenoid (S)
9) Mpep Meningococcal 2B (S)
10) Mpep $CRM_{197}$(S)